# EUROPEAN PATENT APPLICATION

(11) **EP 3 964 580 A1**
(43) Date of publication of application: **09.03.2022**
(21) Application number: 20798494.9
(22) Date of filing: 29.04.2020
(51) Int. Cl.: C12N 15/11, C12Q 1/6886

(54) **TUMOR MARKER STAMP-EP9 BASED ON METHYLATION MODIFICATION AND APPLICATION THEREOF**

(30) Priority: 30.04.2019 CN 201910362192
(71) Applicant: Shanghai Epiprobe Biotechnology Co., Ltd., Shanghai 200233 (CN)
(72) Inventor: LI, Zhenyan, Shanghai 200233 (CN); DONG, Shihua, Shanghai 200233 (CN)
(74) Representative: Office Kirkpatrick
(86) International application number: PCT/CN2020/087886
(87) International publication number: WO 2020/221316

(57) **Abstract**

Provided are an isolated polynucleotide and an application thereof. The isolated polynucleotide is a polynucleotide shown in SEQ ID NO:1 or a fragment or complementary nucleic acid thereof with a modified CpG site. Further provided are an application of the polynucleotide for preparation of a tumor detection reagent or kit, a preparation method therefor, a reagent and an application thereof, a kit, and a method for in vitro detection of the methylome of a sample.

## Description

### Technical field

The disclosure is in the field of markers for diagnosing disease. More specifically, the disclosure relates to a methylation based tumor marker STAMP, Specific Tumor Aligned Methylation of Pan-cancer, and use thereof.

### BACKGROUND OF DISCLOSURE

Tumors have been considered a genetic disease for decades. Several large-scale systematic sequencings for human have confirmed that the number of somatic mutations in cancer tissues is significantly less than expected. These results suggest that cancer is not a simple genetic disease.

In order to diagnose tumor, many new tumor markers have been discovered and used for clinical diagnosis in recent years. Before 1980, tumor markers were mainly hormones, enzymes, proteins and other cell secretions, such as carcinoembryonic antigen (CEA) and alpha fetoprotein (AFP) used as markers of gastric cancer, liver cancer and other tumors, carbohydrate antigen 125 (CA125) used as a marker of cervical cancer, and prostate specific antigen (PSA) used as a marker of prostate cancer. Although these tumor markers are still used in clinic, their sensitivity and accuracy have been difficult to meet the clinical needs.

More and more evidences show that small changes in epigenetic regulation play an important role in tumors. Epigenetics is a subject that studies that the heritable change of gene function without a change of DNA sequence, which eventually leads to the change of phenotype. Epigenetics mainly includes DNA methylation, histone modification, changes of microRNA level and other biochemical processes. DNA methylation is one of the epigenetic mechanisms, refers to the process of transferring methyl from S-adenosylmethionine (methyl donor) to specific bases under the catalysis of DNA methyltransferase. However, DNA methylation in mammals mainly occurs at the C of 5'-CpG-3', which results in 5-methylcytosine (5mC).

Fluid biopsy is a technique for the diagnosis and prediction of tumors using circulating tumor cells or circulating tumor DNA as detection targets. The technology has many shortcomings. First, the sensitivity and specificity are not good enough. The tumor itself is heterogeneous, including a variety of subtypes of cell populations. The proportion of tumor DNA in clinical samples, especially blood samples, is very low. The existing tumor markers are difficult to meet the sensitivity of clinical requirements, and it is easy to cause misdiagnosis. Second, one marker has good effect only for one or a few kinds of tumors. As the DNA sources in blood are very complex, the existing tumor markers cannot solve the complex problems of tumor source and metastasis. Because of these complexities, it is difficult for many DNA methylation tumor markers to have a unified standard in clinical application, which seriously affects the sensitivity and accuracy of the markers.

In the previous study of the inventor, some tumor markers based on methylation modification were found. However, to provide more ways for tumor diagnosis, it is still necessary to find more new tumor markers.

### SUMMARY OF DISCLOSURE

The object of the disclosure is to provide a method for detecting tumor based on abnormal hypermethylation of specific sites in tumor using DNA methylation modification as a tumor marker.

The first aspect of the present disclosure provides an isolated polynucleotide, including: (a) a polynucleotide with a nucleotide sequence as shown in SEQ ID NO: 1; (b) a fragment of the polynucleotide of (a), having at least one (such as 2-119, more specifically 3, 5, 10, 15, 20, 25, 30, 40, 50, 60, 70, 90, 110, 115) modified CpG site; and/or (c) a nucleic acid (such as the polynucleotide with a nucleotide sequence as shown in SEQ ID No: 3) complementary to the polynucleotide or fragment of (a) or (b).

In a preferable embodiment, the modification includes 5-methylation(5mC), 5-hydroxymethylation(5hmC), 5-formylcytosine(5fC) or 5-carboxylcytosine(5-caC).

In a preferable embodiment, for (b), the fragment of the polynucleotide contains bases at positions 786 to 802 (including methylation sites 067 to 070); positions 1 to 325 (including methylation sites 001 to 036) of SEQ ID NO: 1 or 2; positions 362 to 802 (including methylation sites 037 to 070) of SEQ ID NO: 1 or 2; or positions 830 to 1316 (including methylation sites 071 to 119) of SEQ ID NO: 1 or 2.

The second aspect of the disclosure provides an isolated polynucleotide, which is converted from the polynucleotide, and as compared with the sequence of the first aspect, its cytosine C of the CpG site(s) with modification is unchanged, and the unmodified cytosine is converted into T or U.

In a preferable embodiment, it is converted from the polynucleotide corresponding to the first aspect by bisulfite treatment. In another preferable embodiment, the polynucleotide includes: (d) a polynucleotide with a nucleotide sequence as shown in SEQ ID NO: 2 or 4; (e) a fragment of the polynucleotide of (d), having at least one (such as 2-119, more specifically 3, 5, 10, 15, 20, 25, 30, 40, 50, 60, 70, 90, 110, 115) CpG site with modification.

In a preferable embodiment, for (e), the fragment of the polynucleotide contains bases at: positions 786 to 802 (including methylation sites 067 to 070); positions 1 to 325 (including methylation sites 001 to 036) of SEQ ID NO: 1 or 2; positions 362 to 802 (including methylation sites 037 to 070) of SEQ ID NO: 1 or 2; or positions 830 to 1316 (including methylation sites 071 to 119) of SEQ ID NO: 1 or 2; positions 515 to 531 (including methylation sites 067 to 070) of SEQ ID NO: 4 or 3; positions 992 to 1316 (including methylation sites 001 to 036) of SEQ ID NO: 4 or 3; positions 514 to 955 (including methylation sites 037 to 070) of SEQ ID NO: 4 or 3; or positions 1 to 487 (including methylation sites 071 to 119) of SEQ ID NO: 4 or 3.

The third aspect of the disclosure provides a use of the polynucleotide described in the first or second aspect in manufacture of a tumor detection agent or kit.

In a preferable embodiment, the tumors include (but are not limited to): hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer (cancer of the esophagus), gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

In another preferable embodiment, samples of the tumor include but are not limited to: tissue samples, paraffin embedded samples, blood samples, pleural effusion samples, and alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples.

The fourth aspect of the disclosure provides a method of preparing a tumor detection agent, including: providing the polynucleotide described in the first or second aspect, designing a detection agent for specifically detecting the modification on CPG site(s) of a target sequence which is the full length or fragment of the polynucleotide; wherein, the target sequence has at least one (such as 2-119, more specifically, 3, 5, 10, 15, 20, 25, 30, 40, 50, 60, 70, 90, 110, 115) modified CpG site; preferably, the detection agent includes (but is not limited to) primers or probes.

The fifth aspect of the disclosure provides an agent or a combination of agents which specifically detect the modification on CPG site(s) of a target sequence, which is the full length or fragment of any of the polynucleotides described in the first or second aspect and has at least one (such as 2-119, more specifically, 3, 5, 10, 15, 20, 25, 30, 40, 50, 60, 70, 90, 110, 115) modified CpG site.

In a preferable embodiment, the agent or combination of agents is for a gene sequence containing the target sequence (designed based on the gene sequence), and the gene sequence includes gene Panels or gene groups.

In another preferable embodiment, the detection agent comprises: primers or probes.

In another preferable embodiment, the primers are: the primers shown in SEQ ID NO: 3 and 4; the primers shown in SEQ ID NO: 7 and 8, the primers shown in SEQ ID NO: 9 and 10, or the primers shown in SEQ ID NO: 11 and 12.

In the sixth aspect of the disclosure, a use of the agent or combination of agents described in the fifth aspect of the disclosure in the manufacture of a kit for detecting tumors is provided; preferably, the tumors include (but are not limited to): hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

The seventh aspect of the present disclosure provides a detection kit, comprising container(s) and the agent or combination of agents described above in the container(s); preferably, each agent is located in an independent container.

In a preferable embodiment, the kit also includes: bisulfite, DNA purification agent, DNA extraction agent, PCR amplification agent and/or instruction for use (indicating operation steps of the detection and a result judgment standard).

In the eighth aspect of the disclosure, a method for detecting the methylation profile of a sample *in vitro* is provided, including: (i) providing the sample and extracting the nucleic acid; (ii) detecting the modification on CPG site(s) of a target sequence in the nucleic acid of (i), wherein the target sequence is the polynucleotide described in the first aspect or the polynucleotide converted therefrom as described in the second aspect.

In a preferable embodiment, in step (3), the analysis methods include pyrosequencing, bisulfite conversion sequencing, method using methylation chip, qPCR, digital PCR, second generation sequencing, third generation sequencing, whole genome methylation sequencing, DNA enrichment detection, simplified bisulfite sequencing technology, HPLC, MassArray, methylation specific PCR (MSP), or their combination, as well as *in vitro* detection and *in vivo* tracer detection for the combined gene group of partial or all of the methylation sites in the sequence shown in SEQ ID NO: 1. In addition, other methylation detection methods and newly developed methylation detection methods in the future can be applied to the disclosure.

In another preferable embodiment, step (ii) includes: (1) treating the product of (i) to convert the unmodified cytosine into uracil; preferably, the modification includes 5-methylation, 5-hydroxymethylation, 5-formylcytosine or 5-carboxylcytosine; preferably, treating the nucleic acid of step (i) with bisulfite; and (2) analyzing the modification of the target sequence in the nucleic acid treated by (1).

In another preferable embodiment, the abnormal methylation profile is the high level of methylation of C in CPG(s) of the polynucleotide.

In another preferable embodiment, the methylation profile detecting method is not for the purpose of directly obtaining the diagnosis result of a disease, or is not a diagnostic method.

The ninth aspect of the disclosure provides a tumor diagnosis kit, including primer pairs designed based on the sequence described in the first or second aspect of the disclosure, and gene Panels or gene groups containing the sequence, to obtain the characteristics of normal cells and tumor cells through DNA methylation detection.

Other aspects of the disclosure will be apparent to those skilled in the art based on the disclosure herein.

### BRIEF DESCRIPTION OF DRAWINGS

Figure 1, BSP assay for methylation levels of the methylation sites 001 to 036 in SEQ ID NO:1 region in lung cancer cells and normal lung cells. Dark squares indicate "methylated".
Figure 2, BSP assay for methylation levels of the methylation sites 037 to 070 in SEQ ID NO:1 region in lung cancer cells and normal lung cells. Dark squares indicate "methylated".
Figure 3, BSP assay for methylation levels of the methylation sites 071 to 119 in SEQ ID NO:1 region in lung cancer cells and normal lung cells. Dark squares indicate "methylated".
Figure 4, comparison of methylation values of STAMP-EP9 between non-cancer tissues and cancer tissues for clinical samples of leukemia.
Figure 5, comparison of methylation values of STAMP-EP9 between paracancerous samples and cancer tissue samples for clinical samples of breast cancer.
Figure 6, comparison of methylation values of STAMP-EP9 between paracancerous samples and cancer tissue samples for clinical samples of colorectal cancer.
Figure 7, comparison of methylation values of STAMP-EP9 between paracancerous samples and cancer tissue samples for clinical samples of liver cancer.
Figure 8, comparison of methylation values of STAMP-EP9 between paracancerous samples and cancer tissue samples for clinical samples of lung cancer.
Figure 9, comparison of methylation values of STAMP-EP9 between paracancerous samples and cancer tissue samples for clinical samples of pancreatic cancer.

### DETAILED DESCRIPTION

The inventor is committed to the research of tumor markers. After extensive research and screening, the inventor provides a universal DNA methylation tumor marker, STAMP (Specific Tumor Aligned Methylation of Pan-cancer). In normal tissues, STAMP was hypomethylated, while in tumor tissues, it was hypermethylated. It can be used for clinical tumor detection and as the basis of designing tumor diagnostic agents.

### Terms

As used herein, "isolated" refers to a material separated from its original environment (if the material is a natural material, the original environment is the natural environment). For example, in living cells, polynucleotides and polypeptides in their natural state are not isolated or purified, but the same polynucleotides or polypeptides will be isolated ones if they are separated from other substances existed in the natural state.

As used herein, "sample" includes substances suitable for DNA methylation detection obtained from any individual or isolated tissue, cell or body fluid (such as plasma). For example, the samples include but are not limited to: tissue samples, paraffin embedded samples, blood samples, pleural effusion samples, and alveolar lavage fluid samples, ascites and lavage fluid samples, bile samples, stool samples, urine samples, saliva samples, cerebrospinal fluid samples, cell smear samples, cervical scraping or brushing samples, tissue and cell biopsy samples.

As used herein, "hypermethylation" refers to high level of methylation, hydroxymethylation, formylcytosine or carboxylcytosine of CpG in a gene sequence. For example, in the case of methylation specific PCR (MSP), if the PCR reaction with methylation specific primers has positive PCR results, the DNA (gene) region of interest is in hypermethylation state. For another example, in the case of real-time quantitative methylation specific PCR, hypermethylation can be determined based on statistic difference of the methylation status value as compared with the control sample.

### Gene marker

In order to find a useful target for tumor diagnosis, the inventor has identified the target of STAMP-EP9 after extensive and in-depth research. The methylation status with a nucleotide sequence as of STAMP-EP9 gene is significantly different between tumor tissues and non-tumor tissues. If the abnormal hypermethylation with a nucleotide sequence as of STAMP-EP9 gene of a subject is detected, the subject can be identified as having a high-risk of tumor. Moreover, the significant difference of STAMP-EP9 between tumor and non-tumor tissues exists in different kinds of tumors, including solid tumors and non-solid tumors.

Therefore, the disclosure provides an isolated polynucleotide, comprising the nucleotide sequence shown in the sequence of SEQ ID NO: 1 or SEQ ID NO: 3 (the reverse complementary sequence of SEQ ID NO: 1). For tumor cells, the polynucleotide contains 5-methylcytosine (5mC) at C positions of many 5'-CpG-3'. The disclosure also comprises a fragment of the polynucleotide with a nucleotide sequence as shown in SEQ ID NO: 1 or 3, having at least one (such as 2-119, more specifically 3, 5, 10, 15, 20, 25, 30, 40, 50, 60, 70, 90, 110, 115) methylated CpG site. The above polynucleotides or fragments can also be used in the design of detection agents or detection kits.

In some specific embodiments of the disclosure, the fragment of the polynucleotide are, for example, a fragment containing the residues 786-802 (containing CpG sites 017-020) of SEQ ID NO: 1 or 2. Antisense strands of the above fragments are suitable for use in the disclosure. These fragments are merely examples of preferable embodiments of the present disclosure. Based on the information provided by the present disclosure, other fragments can also be selected.

In addition, gene Panels or gene groups containing the sequence shown in the SEQ ID NO: 1 or SEQ ID NO: 2 or a fragment thereof are also encompassed by the disclosure. For the gene Panel or gene group, the characteristics of normal cells and tumor cells can also be identified through DNA methylation detection.

The above polynucleotides can be used as the key regions for analysis of the methylation status in the genome. Their methylation status can be analyzed by various technologies known in the art. Any technique that can be used to analyze the methylation state can be applied to the present disclosure.

When treated with bisulfite, un-methylated cytosine(s) of the above polynucleotides will be converted into uracil, while methylated cytosine(s) remained unchanged.

Therefore, the disclosure also provides the polynucleotides obtained from the above polynucleotides (including the complementary chain (antisense chain)) after being treated with bisulfite, including the polynucleotides with a nucleotide sequence as shown in SEQ ID NO: 2 or SEQ ID NO: 4. These polynucleotides can also be used in the design of detection agents or detection kits.

The disclosure also comprises a fragment of the polynucleotides obtained from the above polynucleotides or the antisense strand thereof after being treated with bisulfite, wherein the fragment contains at least one (such as 2-119, more specifically 3, 5, 10, 15, 20, 25, 30, 40, 50, 60, 70, 90, 110, 115) methylated CpG site. The No. of each CpG site in the antisense strand corresponding to the number of the sense strand is readily obtained according to the content described by the present disclosure.

### Detection agents and kits

Based on the new discovery of the disclosure, a detection agent designed based on said polynucleotide(s) is also provided for detecting the methylation profile of polynucleotide(s) in the sample *in vitro.* The detection methods and agents known in the art for determining the sequence, variation and methylation of the genome can be applied in the disclosure.

Therefore, the disclosure provides a method of preparing a tumor detection agent, including: providing the polynucleotide, designing a detection agent for specifically detecting a target sequence which is the full length or fragment of the polynucleotide; wherein, the target sequence has at least one methylated CpG site.

The detection agent herein includes but is not limited to: primers or probes, etc.

For example, the agent is a primer pair. Based on the sequence of the polynucleotide, those skilled in the art know how to design primer(s). The two primers are on each side of the specific sequence of the target gene to be amplified (including CpG sequence, for the gene region originally methylated, the primer is complementary with CpG, and for the gene region originally un-methylated, the primer is complementary with TpG). It should be understood that based on the new discovery of the disclosure, those skilled in the art can design a variety of primers or probes or other types of detection agents for CpG sites at different positions on the target sequence or their combinations. These primers or probes or other types of detection agents should be included in the technical solution of the disclosure. In a preferable example of the disclosure, the primers are: the primers shown in SEQ ID NO: 5 and 6, which could be used to amplify a product containing CpG sites 001-036 of SEQ ID NO: 1; the primers shown in SEQ ID NO: 7 and 8, which could be used to amplify a product containing CpG sites 037-070 of SEQ ID NO: 1; the primers shown in SEQ ID NO: 9 and 10, which could be used to amplify a product containing CpG sites 071-119 of SEQ ID NO: 1; or the primers shown in SEQ ID NO: 11 and 12, which could be used to amplify a product containing CpG sites 067-070 of SEQ ID NO: 1.

The agent can also be a combination of agents (primer combination), including more than one set of primers, so that the multiple polynucleotides can be amplified respectively.

The disclosure also provides a kit for detecting the methylation profile of polynucleotide in a sample *in vitro,* which comprises container(s) and the above primer pair(s) in the container(s).

In addition, the kit can also include various reagents required for DNA extraction, DNA purification, PCR amplification, etc.

In addition, the kit can also include an instruction for use, which indicates operation steps of the detection and a result judgment standard, for the application of those skilled in the art.

### Detection method

The methylation profile of a polynucleotide can be determined by any technique in the art (such as methylation specific PCR (MSP) or real-time quantitative methylation specific PCR, Methylight), or other techniques that are still developing and will be developed.

Quantitative methylation specific PCR (QMSP) can also be used to detect methylation level. It is a continuous optical monitoring method based on fluorescent PCR, which is more sensitive than MSP. It has high throughput and avoids electrophoresis based result analysis.

Other available technologies include conventional methods in the art such as pyrosequencing, bisulfite converstion sequencing, qPCR, second generation sequencing, whole genome methylation sequencing, DNA enrichment detection, simplified bisulfite sequencing or HPLC, and combined gene group detection. It should be understood that, on the basis of the new disclosure herein, these well-known technologies and some technologies to be developed in the art can be applied to the present disclosure.

As a preferable embodiment of the disclosure, a method of detecting the methylation profile of a polynucleotide in a sample *in vitro* is also provided. The method is based on the follow principle: the un-methylated cytosine can be converted into uracil by bisulfite, which can be transformed into thymine in the subsequent PCR amplification process, while the methylated cytosine remains unchanged; therefore, after the polynucleotide is treated by bisulfite, the methylated site presents a polynucleotide polymorphism (SNP) similar to C/T. Based on the above principle, methylated and un-methylated cytosine can be distinguished by identifying the methylation profile of a polynucleotide in the sample.

The method of the disclosure includes: (a) providing samples and extracting genomic DNA; (b) treating the genomic DNA of step (a) with bisulfite, so as to convert the un-methylated cytosine in the genomic DNA into uracil; (c) analyzing whether the genomic DNA treated in step (b) contains an abnormal methylation profile.

The method of the disclosure can be used for: (i) analyzing whether a subject has tumor by detecting the sample of the subject; (ii) identifying a population having high-risk of tumor. The method needs not to be aimed at obtaining direct diagnosis results.

In a preferable embodiment of the disclosure, DNA methylation is detected by PCR amplification and pyrosequencing. It should be understood by those in the art that DNA methylation detection is not limited to these methods, and any other DNA methylation detection method can be used. The primers used in PCR amplification are not limited to those provided in Examples.

Because of bisulfite treatment, in which un-methylated cytosine in genomic DNA are converted into uracil and then transformed into thymine in the subsequent PCR process, the sequence complexity of the genome will be reduced, and it will be more difficult to amplify specific target fragments by PCR. Therefore, in order to improve amplification efficiency and specificity, nested PCR may be preferable, wherein two sets of primers (outer primers and inner primers) are used in two successive runs of PCR, and the product from the first run undergoes a second run with the second set of primers. However, it should be understood that the detection methods suitable for the present disclosure are not limited thereto.

After the research and assay on clinical samples, the method of the disclosure provides very high accuracy in the clinical diagnosis of tumors. The disclosure can be applied to the fields of tumor auxiliary diagnosis, efficacy evaluation, prognosis monitoring, etc., thus has a high clinical value.

The disclosure is further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Example 1. Nucleic acid sequence for STAMP-EP9 detection

Based on research and screening, the sequence of the STAMP-EP9 tumor marker is provided as follows: SEQ ID NO: 1 (chr20:37356044-37357359, Human/hg19), in which the underlined bases are methylated CpG sites, and each number below the underline indicates the site number.

CGTTGCCAAGTTCGCTGAGCGTCCGCGTCTGGTTGCCTCTCCGCCCCACA
1 2 3 4 5 6
GGGCATGTTCGTGCTGGGCCTACCCTACGCCATCCTGCACGGCGGCTACC
7 8 9 10
TGGGGTTGTTTCTCATCATCTTCGCCGCCGTTGTGTGCTGCTACACCGGCA
11 12 13 14
AGATCCTCATCGCGTGCCTGTACGAGGAGAATGAAGACGGCGAGGTGGT
15 16 17 18 19
GCGCGTGCGGGACTCGTACGTGGCCATAGCCAACGCCTGCTGCGCCCCG
20 21 22 23 24 25 26 27
CGCTTCCCAACGCTGGGCGGCCGAGTGGTGAACGTAGCGCAGATCATCG
28 29 30 31 32 33 34
AGCTGGTGATGACGTGCATCCTGTACGTGGTGGTGAGTGGCAACCTCATG
35 36
TACAACAGCTTCCCGGGGCTGCCCGTGTCGCAGAAGTCCTGGTCCATTAT
37 38 39
CGCCACGGCCGTGCTGCTGCCTTGCGCCTTCCTTAAGAACCTCAAGGCCG
40 41 42 43 44
TGTCCAAGTTCAGTCTGCTGTGCACTCTGGCCCACTTCGTCATCAATATCC
45
TGGTCATAGCCTACTGTCTATCGCGGGCGCGCGACTGGGCCTGGGAGAAG
46 47 48 49 50
GTCAAGTTCTACATCGACGTCAAGAAGTTCCCCATCTCCATTGGCATCAT
51 52
CGTGTTCAGCTACACGTCTCAGATCTTCCTGCCTTCGCTGGAGGGCAATAT
53 54 55
GCAGCAGCCCAGCGAGTTCCACTGCATGATGAACTGGACGCACATCGCA
56 57 58
GCCTGCGTGCTCAAGGGCCTCTTCGCGCTCGTCGCCTACCTCACCTGGGC
59 60 61 62 63
CGACGAGACCAAGGAGGTCATCACGGATAACCTGCCCGGCTCCATCCG
64 65 66 67 68
CGCCGTGGTCAACATCTTTCTGGTGGCCAAGGCGCTGTTGTCCTATCCTCT
69 70 71
GCCATTCTTTGCCGCTGTCGAGGTGCTGGAGAAGTCGCTCTTCCAGGAAG
72 73 74
GCAGCCGCGCCTTTTTCCCGGCCTGCTACAGCGGCGACGGGCGCCTGAAG
75 76 77 78 79 80 81

TCCTGGGGGCTGACGCTGCGCTGCGCGCTCGTCGTCTTCACGCTGCTCAT
82 83 84 85 86 87 88
GGCCATTTATGTGCCGCACTTCGCGCTGCTCATGGGCCTCACCGGCAGCC
89 90 91 92
TCACGGGCGCCGGCCTCTGTTTCTTGCTGCCCAGCCTCTTTCACCTGCGCC
93 94 95 96
TGCTCTGGCGCAAGCTGCTGTGGCACCAAGTCTTCTTCGACGTCGCCATCT
97 98 99 100
TCGTCATCGGCGGCATCTGCAGCGTGTCCGGCTTCGTGCACTCCCTCGAG
101 102 103 104 105 106 107
GGCCTCATCGAAGCCTACCGAACCAACGCGGAGGACTAGGGCGCAAGGG
108 109 110 111 112
CGAGCCCCCGCCGCGCTTCTGCGCTCTCTCCCTTCTCCCCTCACCCCGCCC
113 114 115 116 117 118
CCACCAGCCCAGTGCG
119

The bisulfite treated sequence of SEQ ID NO: 1 is shown in SEQ ID NO: 2 (Y represents C or U) as follows:

YGTTGUUAAGTTYGUTGAGYGTUYGYGTUTGGTTGUUTUTUYGUUUUAUAGGGUATGTT
1 2 3 4 5 6
YGTGUTGGGUUTAUUUTAYGUUATUUTGUAYGGYGGUTAUUTGGGGTTGTTTUTUATUAT
7 8 9 10
UTTYGUYGUYGTTGTGTGUTGUTAUAUYGGUAAGATUUTUATYGYGTGUUTGTAYGAGGA
11 12 13 14 15 16 17
GAATGAAGAYGGYGAGGTGGTGYGYGTGYGGGAUTYGTAYGTGGUUATAGUUAAYGUUT
18 19 20 21 22 23 24 25
GUTGYGUUUYGYGUTTUUUAAYGUTGGGYGGUYGAGTGGTGAAYGTAGYGUAGATUAT
26 27 28 29 30 31 32 33
YGAGUTGGTGATGAYGTGUATUUTGTAYGTGGTGGTGAGTGGUAAUUTUATGTAUAAUAG
34 35 36
UTTUUYGGGGUTGUUYGTGTYGUAGAAGTUUTGGTUUATTATYGUUAYGGUYGTGUTGUT
37 38 39 40 41 42
GUUTTGYGUUTTUUTTAAGAAUUTUAAGGUYGTGTUUAAGTTUAGTUTGUTGTGUAUTUTG
43 44
GUUUAUTTYGTUATUAATATUUTGGTUATAGUUTAUTGTUTATYGYGGGYGYGYGAUTGG
45 46 47 48 49 50
GUUTGGGAGAAGGTUAAGTTUTAUATYGAYGTUAAGAAGTTUUUUATUTUUATTGGUATU
51 52
ATYGTGTTUAGUTAUAYGTUTUAGATUTTUUTGUUTTYGUTGGAGGGUAATATGUAGUAGU
53 54 55
UUAGYGAGTTUUAUTGUATGATGAAUTGGAYGUAUATYGUAGUUTGYGTGUTUAAGGGUU
56 57 58 59
TUTTYGYGUTYGTYGUUTAUUTUAUUTGGGUYGAYGAGAUUAAGGAGGTUATUAYGGATA
60 61 62 63 64 65 66
AUUTGUUYGGUTUUATUYGYGUYGTGGTUAAUATUTTUTGGTGGUUAAGGYGUTGTTGT
67 68 69 70 71
UUTATUUTUTGUUATTUTTTGUYGUTGTYGAGGTGUTGGAGAAGTYGUTUTTUUAGGAAGG
72 73 74
UAGUYGYGUUTTTTTUUYGGUUTGUTAUAGYGGYGAYGGGYGUUTGAAGTUUTGGGGGUT
75 76 77 78 79 80 81
GAYGUTGYGUTGYGYGUTYGTYGTUTTUAYGUTGUTUATGGUUATTTATGTGUYGUAUTT
82 83 84 85 86 87 88 89
YGYGUTGUTUATGGGUUTUAUYGGUAGUUTUAYGGGYGUYGGUUTUTGTTUTTGUTGUU
90 91 92 93 94 95
UAGUUTUTTTUAUUTGYGUUTGUTUTGGYGUAAGUTGUTGTGGUAUUAAGTUTTUTTYGA
96 97 98
YGTYGUUATUTTYGTUATYGGYGGUATUTGUAGYGTGTUYGGUTTYGTGUAUTUUUTYGA
99 100 101 102 103 104 105 106 107
GGGUUTUATYGAAGUUTAUYGAAUUAAYGYGGAGGAUTAGGGYGUAAGGGYGAGUUUU
108 109 110 111 112 113
YGUYGYGUTTUTGYGUTUTUTUUUTTUTUUUTUAUUUYGUUUUUAUUAGUUUAGTGYG
114 115 116 117 118 119

The reverse complementary sequence of SEQ ID NO: 1 is shown in SEQ ID NO: 3 as follows:

CGCACTGGGCTGGTGGGGGCGGGGTGAGGGGAGAAGGAGAGAGCGCAGAAGCGCGGCGGGGGCTCGCCCTT
GCGCCCTAGTCCTCCGCGTTGGTTCGGTAGGCTTCGATGAGGCCCTCGAGGGAGTGCACGAAGCCGGACACGC
TGCAGATGCCGCCGATGACGAAGATGGCGACGTCGAAGAAGACTTGGTGCCACAGCAGCTTGCGCCAGAGCAG
GCGCAGGTGAAAGAGGCTGGGCAGCAAGAAACAGAGGCCGGCGCCCGTGAGGCTGCCGGTGAGGCCCATGAG
CAGCGCGAAGTGCGGCACATAAATGGCCATGAGCAGCGTGAAGACGACGAGCGCGCAGCGCAGCGTCAGCCC
CCAGGACTTCAGGCGCCCGTCGCCGCTGTAGCAGGCCGGGAAAAAGGCGCGGCTGCCTTCCTGGAAGAGCGAC
TTCTCCAGCACCTCGACAGCGGCAAAGAATGGCAGAGGATAGGACAACAGCGCCTTGGCCACCAGAAAGATGT
TGACCACGGCGCGGATGGAGCCGGGCAGGTTATCCGTGATGACCTCCTTGGTCTCGTCGGCCCAGGTGAGGT
AGGCGACGAGCGCGAAGAGGCCCTTGAGCACGCAGGCTGCGATGTGCGTCCAGTTCATCATGCAGTGGAACTC
GCTGGGCTGCTGCATATTGCCCTCCAGCGAAGGCAGGAAGATCTGAGACGTGTAGCTGAACACGATGATGCCA
ATGGAGATGGGGAACTTCTTGACGTCGATGTAGAACTTGACCTTCTCCCAGGCCCAGTCGCGCGCCCGCGATAG
ACAGTAGGCTATGACCAGGATATTGATGACGAAGTGGGCCAGAGTGCACAGCAGACTGAACTTGGACACGGCC
TTGAGGTTCTTAAGGAAGGCGCAAGGCAGCAGCACGGCCGTGGCGATAATGGACCAGGACTTCTGCGACACGG
GCAGCCCCGGGAAGCTGTTGTACATGAGGTTGCCACTCACCACCACGTACAGGATGCACGTCATCACCAGCTC
GATGATCTGCGCTACGTTCACCACTCGGCCGCCCAGCGTTGGGAAGCGCGGGGCGCAGCAGGCGTTGGCTATG
GCCACGTACGAGTCCCGCACGCGCACCACCTCGCCGTCTTCATTCTCCTCGTACAGGCACGCGATGAGGATCTT

GCCGGTGTAGCAGCACACAACGGCGGCGAAGATGATGAGAAACAACCCCAGGTAGCCGCCGTGCAGGATGGC
GTAGGGTAGGCCCAGCACGAACATGCCCTGTGGGGCGGAGAGGCAACCAGACGCGGACGCTCAGCGAACTTG
GCAACG

The bisulfite treated sequence of SEQ ID NO: 3 is shown in SEQ ID NO: 4 (Y represents C or U) as follows:

YGUAUTGGGUTGGTGGGGGYGGGGTGAGGGAGAAGGAGAGAGYGUAGAAGYGYGGYGGGGGUTYGUUUT
TGYGUUUTAGTUUTUYGYGTTGGTTYGGTAGGUTTYGATGAGGUUUTYGAGGGAGTGUAYGAAGUYGGAUAY
GUTGUAGATGUYGUYGATGAYGAAGATGGYGAYGTYGAAGAAGAUTTGGTGUUAUAGUAGUTTGYGUUAGA
GUAGGYGUAGGTGAAAGAGGUTGGGUAGUAAGAAAUAGAGGUYGGYGUUYGTGAGGUTGUYGGTGAGGUU
UATGAGUAGYGYGAAGTGYGGUAUATAAATGGUUATGAGUAGYGTGAAGAYGAYGAGYGYGUAGYGUAGYG
TUAGUUUUUAGGAUTTUAGGYGUUYGTYGUYGUTGTAGUAGGUYGGGAAAAAGGYGYGGUTGUUTTUUTGG
AAGAGYGAUTTUTUUAGUAUUTYGAUAGYGGUAAAGAATGGUAGAGGATAGGAUAAUAGYGUUTTGGUUAU
UAGAAAGATGTTGAUUAYGGYGYGGATGGAGUYGGGUAGGTTATUYGTGATGAUUTUUTTGGTUTYGTYGG
UUUAGGTGAGGTAGGYGAYGAGYGYGAAGAGGUUUTTGAGUAYGUAGGUTGYGATGTGYGTUUAGTTUATU
ATGUAGTGGAAUTYGUTGGGUTGUTGUATATTGUUUTUUAGYGAAGGUAGGAAGATUTGAGAYGTGTAGUTG
AAUAYGATGATGUUAATGGAGATGGGGAAUTTUTTGAYGTYGATGTAGAAUTTGAUUTTUTUUUAGGUUUAG
TYGYGYGUUYGYGATAGAUAGTAGGUTATGAUUAGGATATTGATGAYGAAGTGGGUUAGAGTGUAUAGUAG
AUTGAAUTTGGAUAYGGUUTTGAGGTTUTTAAGGAAGGYGUAAGGUAGUAGUAYGGUYGTGGYGATAATGG
AUUAGGAUTTUTGYGAUAYGGGUAGUUUYGGGAAGUTGTTGTAUATGAGGTTGUUAUTUAUUAUUAYGTAU
AGGATGUAYGTUATUAUUAGUTYGATGATUTGYGUTAYGTTUAUUAUTYGGUYGUUUAGYGTTGGGAAGYGY
GGGGYGUAGUAGGYGTTGGUTATGGUUAYGTAYGAGTUUYGUAYGYGUAUUAUUTYGUYGTUTTUATTUTUU
TYGTAUAGGUAYGYGATGAGGATUTTGUYGGTGTAGUAGUAUAUAAYGGYGGYGAAGATGATGAGAAAUAA
UUUUAGGTAGUYGUYGTGUAGGATGGYGTAGGGTAGGUUUAGUAYGAAUATGUUUTGTGGGGYGGAGAGGU
AAUUAGAYGYGGAYGUTUAGYGAAUTTGGUAAYG

### Example 2. Difference in methylation of STAMP-EP9 CpG sites between tumor cells and non-tumor cells -- Bisulfite Sequencing PCR (BSP)

Bisulfite Sequencing PCR comprises the following steps:
1. Genomic DNA was extracted from lung cancer cell line A549 and normal lung cell line MRC5;
2. The extracted genomic DNA of lung cancer cell line A549 and normal lung cell line MRC5 were treated with bisulfite and used as templates for subsequent PCR amplification;
3. Primers (SEQ ID NO: 5-10; Table 1)were designed according to SEQ ID NO: 1 and used for amplification;
4. After PCR amplification, 2% agarose gel electrophoresis was used to detect the specificity of the PCR fragments. The target fragments were recovered by gel cutting and connected to T vector which was transformed into competent *Escherichia coli.* The bacteria were spread on plate, and clones were selected the next day and sequenced. More than ten clones were selected for each fragment for Sanger sequencing.

**Table 1, BSP primers**

| Primer Name | 5'-3' Primer Sequence | Detected CpG site No. |
|---|---|---|
| STAMP-EP9-Sanger-Primer-F | GATATTYGGGTTTATTATTAGTTTT (SEQ ID NO: 5) | CpG 001-036 of SEQ ID NO: 1 |
| ST AM P-EP9-Sanger-Primer-R | CATAAAATTACCACTCACCACCAC (SEQ ID NO: 6) | |
| STAMP-EP9-Sanger-Primer-F | GTGGTGGTGAGTGGTAATTTTATG (SEQ ID NO: 7) | CpG 037-070 of SEQ ID NO: 1 |
| STAMP-EP9-Sanger-Primer-R | TAACCACCAAAAAAATATTAACCA (SEQ ID NO: 8) | |
| STAMP-EP9-Sanger-Primer-F | TGGTTAATATTTTTTTGGTGGTTA (SEQ ID NO: 9) | CpG 071-119 of SEQ ID NO: 1 |
| ST AM P-EP9-Sanger-Primer-R | ATTTAAAACTTAACCTCCCAAAC (SEQ ID NO: 10) | |

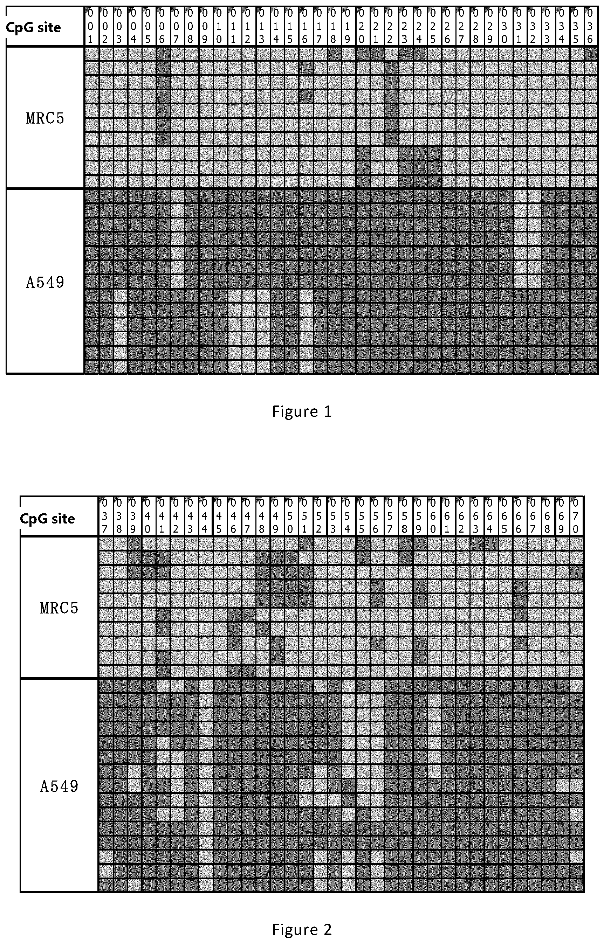

Figure 1 shows BSP assay for methylation levels of the methylation sites 001 to 036 in SEQ ID NO:1 region in lung cancer cells and normal lung cells, which indicates that the methylation level of STAMP-EP9 of lung cancer cells is significantly higher than that of normal lung cells.

Figure 1 shows BSP assay for methylation levels of the methylation sites 037 to 070 in SEQ ID NO:2 region in lung cancer cells and normal lung cells, which indicates that the methylation level of STAMP-EP9 of lung cancer cells is significantly higher than that of normal lung cells.

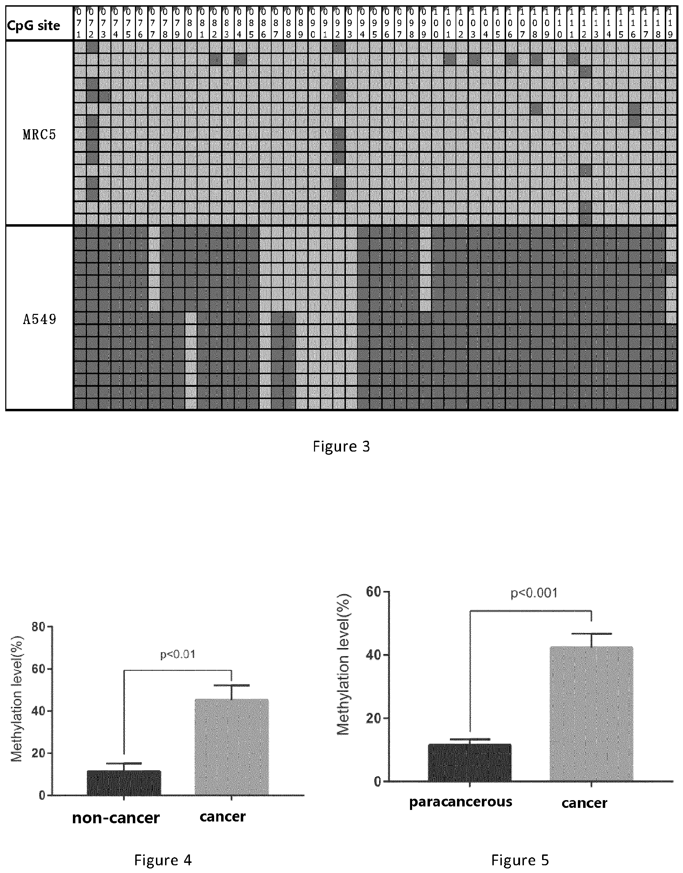

Figure 3 shows BSP assay for methylation levels of the methylation sites 071 to 119 in SEQ ID NO:1 region in lung cancer cells and normal lung cells, which indicates that the methylation level of STAMP-EP9 of lung cancer cells is significantly higher than that of normal lung cells.

### Example 3: Difference in methylation of STAMP-EP9 CpG sites between tumor cells and non-tumor cells-pyrosequencing

Pyrosequencing comprises the following steps:
1. Obtaining of the clinical samples: paracancerous/non-cancer were used as the control group, and cancer tissue samples were used as the experimental group;
2. DNA extraction: DNA was extracted from the experimental group and the control group respectively. Phenol-chloroform extraction method was used in this experiment, which is not limited thereto;
3. Bisulfite treatment: the extracted DNA samples were treated with bisulfite and the procedures were strictly followed. EZ DNA Methylation-Gold Kit (ZYMO Research, Cat# D5006) was used in this experiment, which is not limited thereto;
4. Primer design: PCR primers and pyrosequencing primers were designed according to the characteristics of SEQ ID NO: 1 of STAMP-EP9 sequence. The methylation values of STAMP-EP9 were detected as the methylation values of CpG sites 067-070. The PCR primers sequences, pyrosequencing primers sequences, and the pyrosequencing detecting sequences and the detected sites are shown as SEQ ID NO: 11-14 (Table 2);
5. PCR amplification and agarose gel electrophoresis: The bisulfite treated samples were used as templates for PCR amplification. The specificity of PCR amplification was identified by agarose gel electrophoresis of the amplified products;
6. Pyrosequencing: Pyro Mark Q96 ID pyrosequencing instrument (QIAGEN) was used for sequencing, and the procedures in instructions were strictly followed;
7. Calculation of STAMP-EP9 methylation value: pyrosequencing can detect the methylation value of each site in the target region, respectively, and the average methylation value of all sites were calculated as the STAMP-EP9 methylation value in the sample;
8. Results analysis: the methylation value of STAMP-EP9 was compared between the paracancerous/non-cancer control group and the cancer experimental group.

**Table 2, Pyro-primers**

| Primer Name | 5'-3' sequence | Detected CpG site No. |
|---|---|---|
| STAMP-EP9-Pyroseq-Primer-F | TTTGGGTYGAYGAGATTAAGGAGGTTATTA(SEQ ID NO: 11) | Detect CpG 067-070 of SEQ ID NO: 1 |
| STAMP-EP9-Pyroseq-Primer-R (with 5'-Biotin modification) | CCTTAACCACCRAAAAAATATTAACCAC (SEQ ID NO: 12) | |
| STAMP-EP9-Pyroseq-Primer-Seq | TTAAGGAGGTTATTAYGGATAATTTGTT (SEQ ID NO:13) | |
| pyrosequencing detecting sequences | YGGTTTTATTYGYGTYGTGGTTAATATTTTTTYGG T (SEQ ID NO: 14) | |

### Example 4. leukemia clinical sample validation - pyrosequencing

STAMP-EP9 methylation value between the control group of 8 non-leukemia bone marrow smear clinical samples and the experimental group of 8 leukemia bone marrow smear clinical samples was compared according to the pyrosequencing above.

The result in Figure 4 shows that, in clinical samples of leukemia, the methylation value of STAMP-EP9 in the experimental group was significantly higher than that of non-cancer tissues.

### Example 5. Breast cancer - clinical sample validation - pyrosequencing

STAMP-EP9 methylation value between the control group of 5 cases of breast cancer paracancerous clinical samples and the experimental group of 5 cases of breast cancer clinical samples was compared according to the pyrosequencing above.

The result in Figure 5 shows that, in clinical samples of breast cancer, the methylation value of STAMP-EP9 in the experimental group was significantly higher than that of paracancerous tissues.

### Example 6. Colorectal cancer clinical sample validation - pyrosequencing

STAMP-EP9 methylation value was analyzed on the control group of 8 cases of colorectal cancer paracancerous clinical samples and the experimental group of 8 cases of colorectal cancer clinical samples through pyrosequencing assay.

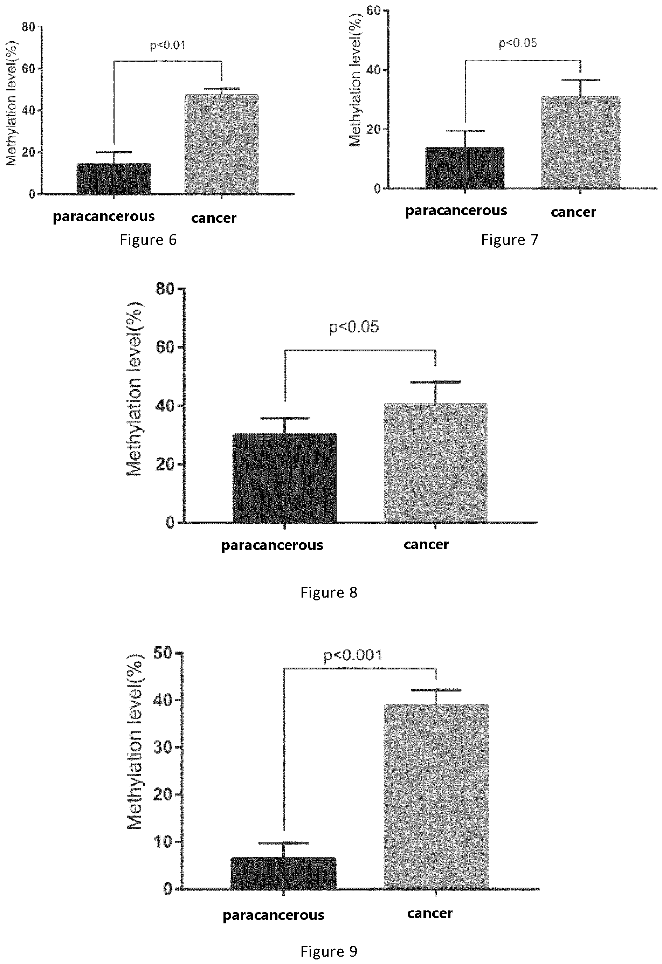

The result in Figure 6 shows that, in clinical samples of colorectal cancer, the methylation value of STAMP-EP9 in the experimental group was significantly higher than that of paracancerous tissues.

### Example 7. Liver cancer clinical sample validation - pyrosequencing

STAMP-EP9 methylation value was analyzed on the control group of 8 cases of liver cancer paracancerous clinical samples and the experimental group of 8 cases of liver cancer clinical samples through pyrosequencing.

The result in Figure 7 shows that, in clinical samples of liver cancer, the methylation value of STAMP-EP9 in the experimental group was significantly higher than that of paracancerous tissues.

### Example 8. Lung cancer clinical sample validation - pyrosequencing

STAMP-EP9 methylation value was analyzed on the control group of 4 cases of lung cancer paracancerous clinical samples and the experimental group of 4 cases of lung cancer clinical samples according to the pyrosequencing.

The result in Figure 8 shows that, in clinical samples of lung cancer, the methylation value of STAMP-EP9 in the experimental group was significantly higher than that of paracancerous tissues.

### Example 9. Pancreatic cancer clinical sample validation - pyrosequencing

STAMP-EP9 methylation value was analyzed on the control group of 4 cases of pancreatic cancer paracancerous clinical samples and the experimental group of 4 cases of pancreatic cancer clinical samples through pyrosequencing.

The result in Figure 9 shows that, in clinical samples of pancreatic cancer, the methylation value of STAMP-EP9 in the experimental group was significantly higher than that of paracancerous tissues.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

Sequence Listing

<110>  SHANGHAI EPIPROBE BIOTECHNOLOGY CO.,LTD.

<120>  TUMOR MARKER STAMP-EP9 BASED ON METHYLATION MODIFICATION AND
APPLICATION THEREOF

<130>  192648PCWO

<150>  201910362192.3
<151>  2020-04-30

<160>  14

<170>  SIPOSequenceListing 1.0

<210>  1
<211>  1316
<212>  DNA
<213>  Homo sapiens

<400>  1
cgttgccaag ttcgctgagc gtccgcgtct ggttgcctct ccgccccaca gggcatgttc      60
gtgctgggcc taccctacgc catcctgcac ggcggctacc tggggttgtt tctcatcatc     120
ttcgccgccg ttgtgtgctg ctacaccggc aagatcctca tcgcgtgcct gtacgaggag     180
aatgaagacg gcgaggtggt gcgcgtgcgg gactcgtacg tggccatagc caacgcctgc     240
tgcgccccgc gcttcccaac gctgggcggc cgagtggtga acgtagcgca gatcatcgag     300
ctggtgatga cgtgcatcct gtacgtggtg gtgagtggca acctcatgta caacagcttc     360
ccggggctgc ccgtgtcgca gaagtcctgg tccattatcg ccacggccgt gctgctgcct     420
tgcgccttcc ttaagaacct caaggccgtg tccaagttca gtctgctgtg cactctggcc     480
cacttcgtca tcaatatcct ggtcatagcc tactgtctat cgcgggcgcg cgactgggcc     540
tgggagaagg tcaagttcta catcgacgtc aagaagttcc ccatctccat tggcatcatc     600
gtgttcagct acacgtctca gatcttcctg ccttcgctgg agggcaatat gcagcagccc     660
agcgagttcc actgcatgat gaactggacg cacatcgcag cctgcgtgct caagggcctc     720
ttcgcgctcg tcgcctacct cacctgggcc gacgagacca aggaggtcat cacggataac     780
ctgcccggct ccatccgcgc cgtggtcaac atctttctgg tggccaaggc gctgttgtcc     840
tatcctctgc cattctttgc cgctgtcgag gtgctggaga agtcgctctt ccaggaaggc     900
agccgcgcct ttttcccggc ctgctacagc ggcgacgggc gcctgaagtc ctgggggctg     960
acgctgcgct gcgcgctcgt cgtcttcacg ctgctcatgg ccatttatgt gccgcacttc    1020
gcgctgctca tgggcctcac cggcagcctc acgggcgccg gcctctgttt cttgctgccc    1080
agcctctttc acctgcgcct gctctggcgc aagctgctgt ggcaccaagt cttcttcgac    1140
gtcgccatct tcgtcatcgg cggcatctgc agcgtgtccg gcttcgtgca ctccctcgag    1200
ggcctcatcg aagcctaccg aaccaacgcg gaggactagg gcgcaagggc gagcccccgc    1260
cgcgcttctg cgctctctcc cttctcccct caccccgccc ccaccagccc agtgcg        1358

<210>  2
<211>  1316
<212>  DNA/RNA
<213>  Artificial Sequence

<220>
<221>  misc_feature
<222>  (1)..(1316)
<223>  y is c or u


<400>  2
ygttguuaag ttygutgagy gtuygygtut ggttguutut uyguuuuaua ggguatgtty      60
gtgutggguu tauuutaygu uatuutguay ggyggutauu tggggttgtt tutuatuatu     120
ttyguyguyg ttgtgtgutg utauauyggu aagatuutua tygygtguut gtaygaggag     180
aatgaagayg gygaggtggt gygygtgygg gautygtayg tgguuatagu uaayguutgu     240
tgyguuuygy guttuuuaay gutgggyggu ygagtggtga aygtagygua gatuatygag     300
utggtgatga ygtguatuut gtaygtggtg gtgagtggua auutuatgta uaauaguttu     360

uyggggutgu uygtgtygua gaagtuutgg tuuattatyg uuaygguygt gutgutguut 420
tgyguuttuu ttaagaauut uaagguygtg tuuaagttua gtutgutgtg uaututgguu 480
uauttygtua tuaatatuut ggtuataguu tautgtutat ygygggygyg ygautggguu 540
tgggagaagg tuaagttuta uatygaygtu aagaagtuuu uuatutuuat tgguatuaty 600
gtgttuagut auaygtutua gatuttuutg uuttygutgg aggguaatat guaguaguuu 660
agygagttuu autguatgat gaautggayg uauatyguag uutgygtgut uaaggguutu 720
ttygygutyg tyguutauut uauutggguy gaygagauua aggaggtuat uayggataau 780
utguuyggut uuatuygygu ygtggtuaau atutttutgg tgguuaaggy gutgttgtuu 840
tatuututgu uattutttgu ygutgtygag gtgutggaga agtygututt uuaggaaggu 900
aguygyguut ttttuuyggu utgutauagy ggygaygggy guutgaagtu utgggggutg 960
aygutgygut gygygutygt ygtuttuayg utgutuatgg uuatttatgt guyguautty 1020
gygutgutua tggguutuau ygguaguutu ayggygyuyg guutuutgttt uttgutguuu 1080
aguututttu auutgyguut gututggygu aagutgutgt gguauuaagt uttuttygay 1140
gtyguuatut tygtuatygg ygguatutgu agygtgtuyg guttygtgua utuuutygag 1200
gguutuatyg aaguutauyg aauuaaygyg gaggautagg gyguaagggy gaguuuuygu 1260
ygyguttutg ygutututuu uttutuuuut uauuuyguuu uuauuaguuu agtgyg 1358

<210> 3
<211> 1316
<212> DNA
<213> Homo sapiens

<400> 3
cgcactgggc tggtgggggc ggggtgaggg gagaagggag agagcgcaga agcgcggcgg 60
gggctcgccc ttgcgcccta gtcctccgcg ttggttcggt aggcttcgat gaggccctcg 120
agggagtgca cgaagccgga cacgctgcag atgccgccga tgacgaagat ggcgacgtcg 180
aagaagactt ggtgccacag cagcttgcgc cagagcaggc gcaggtgaaa gaggctgggc 240
agcaagaaac agaggccggc gcccgtgagg ctgccggtga ggcccatgag cagcgcgaag 300
tgcggcacat aaatggccat gagcagcgtg aagacgacga gcgcgcagcg cagcgtcagc 360
ccccaggact tcaggcgccc gtcgccgctg tagcaggccg ggaaaaaggc gcggctgcct 420
tcctggaaga gcgacttctc cagcacctcg acagcggcaa agaatggcag aggataggac 480
aacagcgcct tggccaccag aaagatgttg accacggcgc ggatggagcc gggcaggtta 540
tccgtgatga cctccttggt ctcgtcggcc caggtgaggt aggcgacgag cgcgaagagg 600
cccttgagca cgcaggctgc gatgtgcgtc cagttcatca tgcagtggaa ctcgctgggc 660
tgctgcatat tgccctccag cgaaggcagg aagatctgag acgtgtagct gaacacgatg 720
atgccaatgg agatggggaa cttcttgacg tcgatgtaga acttgacctt ctcccaggcc 780
cagtcgcgcg cccgcgatag acagtaggct atgaccagga tattgatgac gaagtgggcc 840
agagtgcaca gcagactgaa cttggacacg gccttgaggt tcttaaggaa ggcgcaaggc 900
agcagcacgg ccgtggcgat aatggaccag gacttctgcg acacgggcag ccccgggaag 960
ctgttgtaca tgaggttgcc actcaccacc acgtacagga tgcacgtcat caccagctcg 1020
atgatctgcg ctacgttcac cactcggccg cccagcgttg ggaagcgcgg ggcgcagcag 1080
gcgttggcta tggccacgta cgagtcccgc acgcgcacca cctcgccgtc ttcattctcc 1140
tcgtacaggc acgcgatgag gatcttgccg gtgtagcagc acacaacggc ggcgaagatg 1200
atgagaaaca accccaggta gccgccgtgc aggatggcgt agggtaggcc cagcacgaac 1260
atgccctgtg gggcggagag gcaaccagac gcggacgctc agcgaacttg gcaacg 1358

<210> 4
<211> 1316
<212> DNA/RNA
<213> Artificial Sequence

<220>
<221> misc_feature
<222> (1)..(1316)

<400> 4
yguautgggu tggtgggggy ggggtgaggg gagaagggag agagyguaga agygyggygg 60
gggutyguuu ttgyguuuta gtuutuygyg ttggttyggt agguttygat gagguuutyg 120
agggagtgua ygaaguygga uaygutguag atguyguyga tgaygaagat ggygaygtyg 180
aagaagautt ggtguuauag uaguttgygu uagaguaggy guaggtgaaa gaggutgggu 240
aguaagaaau agagguyggy guuygtgagg utguyggtga gguuuatgag uagygygaag 300
tgygguauat aaatgguuat gaguagygtg aagaygayga gygyguagyg uagygtuagu 360
uuuuaggaut tuaggyguuy gtyguygutg taguagguyg ggaaaaaggy gyggutguut 420

tuutggaaga gygauttutu uaguauutyg auagygguaa agaatgguag aggataggau 480
aauagyguut tgguuauuag aaagatgttg auuayggygy ggatggaguy ggguaggtta 540
tuygtgatga uutuuttggt utygtygguu uaggtgaggt aggygaygag ygygaagagg 600
uuuttgagua yguaggutgy gatgtgygtu uagttuatua tguagtggaa utygutgggu 660
tgutguatat tguuutuuag ygaagguagg aagatutgag aygtgtagut gaauaygatg 720
atguuaatgg agatggggaa uttuttgayg tygatgtaga auttgauutt utuuuagguu 780
uagtygygyg uuygygatag auagtaggut atgauuagga tattgatgay gaagtggguu 840
agagtguaua guagautgaa uttggauayg guuttgaggt tuttaaggaa ggyguaaggu 900
aguaguaygg uygtggygat aatggauuag gauttutgyg auayggguag uuuygggaag 960
utgttgtaua tgaggttguu autuauuauu aygtauagga tguaygtuat uauuagutyg 1020
atgatutgyg utaygttuau uautygguyg uuuagygttg ggaagygygg ggyguaguag 1080
gygttgguta tgguuaygta ygagtuuygu aygyguauua uutyguygtu ttuattutuu 1140
tygtauaggu aygygatgag gatuttguyg gtgtaguagu auauaayggy ggygaagatg 1200
atgagaaaua auuuuaggta guyguygtgu aggatggygt agggtagguu uaguaygaau 1260
atguuutgtg gggyggagag guaauuagay gyggaygutu agygaauttg guaayg 1358

<210> 5
<211> 25
<212> DNA/RNA
<213> Primer

<400> 5
gatattyggg tttattatta gtttt 25

<210> 6
<211> 24
<212> DNA/RNA
<213> Primer

<400> 6
cataaaatta ccactcacca ccac 24

<210> 7
<211> 24
<212> DNA/RNA
<213> Primer

<400> 7
gtggtggtga gtggtaattt tatg 24

<210> 8
<211> 24
<212> DNA/RNA
<213> Primer

<400> 8
taaccaccaa aaaaatatta acca 24

<210> 9
<211> 24
<212> DNA/RNA
<213> Primer

<400> 9
tggttaatat ttttttggtg gtta 24

<210> 10
<211> 23
<212> DNA/RNA
<213> Primer

<400> 10
atttaaaact taacctccca aac 23

<210>  11
<211>  30
<212>  DNA/RNA
<213>  Primer

<400>  11
tttgggtyga ygagattaag gaggttatta                                        30

<210>  12
<211>  28
<212>  DNA/RNA
<213>  Primer

<400>  12
   ccttaaccac craaaaaata ttaaccac                                           28

<210>  13
<211>  28
<212>  DNA/RNA
<213>  Primer

<400>  13
ttaaggaggt tattayggat aatttgtt                                          28

<210>  14
<211>  36
<212>  DNA/RNA
<213>  Primer

<400>  14
yggttttatt ygygtygtgg ttaatatttt ttyggt                                 36

## Claims

1. An isolated polynucleotide, wherein the isolated polynucleotide comprises:
(a) a polynucleotide with a nucleotide sequence as shown in SEQ ID NO: 1;
(b) a fragment of the polynucleotide of (a), having at least one CpG site with modification; and/or
(c) a nucleic acid complementary to the polynucleotide or fragment of (a) or (b).

2. The isolated polynucleotide according to claim 1, wherein the modification comprises 5-methylation, 5-hydroxymethylation, 5-formylcytosine or 5-carboxylcytosine.

3. The isolated polynucleotide according to claim 1, wherein, for (b), the fragment of the polynucleotide contains:
bases at positions 786 to 802 of SEQ ID NO: 1 or 2;
bases at positions 1 to 325 of SEQ ID NO: 1 or 2;
bases at positions 362 to 802 of SEQ ID NO: 1 or 2;
bases at positions 1 to 1316 of SEQ ID NO: 1 or 2.

4. An isolated polynucleotide, wherein, the polynucleotide is converted from the polynucleotide according to claim 1 or 2 or 3, and as compared with the sequence in claim 1, the cytosine C of the CpG site(s) with modification is unchanged, and the unmodified cytosine is converted into T or U.

5. The polynucleotide according to claim 4, wherein the polynucleotide comprises:
(d) a polynucleotide with a nucleotide sequence as shown in SEQ ID NO: 2 or 4;
(e) a fragment of the polynucleotide of (d), having at least one CpG site with modification.

6. The polynucleotide according to claim 5, wherein, for (e), the fragment of the polynucleotide contains:
bases at positions 786 to 802 of SEQ ID NO: 1 or 2;
bases at positions 1 to 325 of SEQ ID NO: 1 or 2;
bases at positions 362 to 802 of SEQ ID NO: 1 or 2;
bases at positions 830 to 1316 of SEQ ID NO: 1 or 2;
bases at positions 515 to 531 of SEQ ID NO: 4 or 3;
bases at positions 992 to 1316 of SEQ ID NO: 4 or 3;
bases at positions 514 to 955 of SEQ ID NO: 4 or 3; or
bases at positions 1 to 487 of SEQ ID NO: 4 or 3.

7. Use of the polynucleotide according to any of claims 1-6 in manufacture of a tumor detection agent or kit.

8. The use according to claim 7, wherein, the tumor comprises: hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

9. The use according to claim 7 or 8, wherein samples of the tumor comprise: tissue samples, paraffin embedded samples, blood samples, pleural effusion samples, and alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples.

10. A method of preparing a tumor detection agent, comprising: providing the polynucleotide according to any of claims 1-6, designing a detection agent for specifically detecting a target sequence which is the full length or fragment of the polynucleotide; wherein, the target sequence has at least one modified CpG site; preferably, the detection agent comprises: primers or probes.

11. An agent or a combination of agents, wherein, the agent or the combination of agents specifically detect the modification on CPG site(s) of a target sequence, which is the full length or fragment of the polynucleotides according to any of claims 1-6 and has at least one modified CpG site.

12. The agent or the combination of agents according to claim 11, wherein the agent or combination of agents is for a gene sequence containing the target sequence, and the gene sequence comprises gene Panels or gene groups.

13. The agent or the combination of agents according to claim 11, wherein the agent or combination of agents comprise: primers or probes; preferably, the primers are:
primers shown in SEQ ID NO: 5 and 6;
primers shown in SEQ ID NO: 7 and 8;
primers shown in SEQ ID NO: 9 and 10; or
primers shown in SEQ ID NO: 11 and 12.

14. Use of the agent or combination of agents according to any of claims 11-13 in the manufacture of a kit for detecting tumors; preferably, the tumors comprise: hematologic cancers such as leukemia, lymphoma, multiple myeloma; gynecological and reproductive system tumors such as breast cancer, ovarian cancer, cervical cancer, vulvar cancer, testicular cancer, prostate cancer, penile cancer; digestive system tumors such as esophageal cancer, gastric cancer, colorectal cancer, liver cancer, pancreatic cancer, bile duct and gallbladder cancer; respiratory system tumors such as lung cancer, pleuroma; nervous system tumors such as glioma, neuroblastoma, meningioma; head and neck tumors such as oral cancer, tongue cancer, laryngeal cancer, nasopharyngeal cancer; urinary system tumors such as kidney cancer, bladder cancer, skin and other systems tumors such as skin cancer, melanoma, osteosarcoma, liposarcoma, thyroid cancer.

15. A detection kit, comprising:
container(s) and the agent or combination of agents according to any of claims 11-13 in the container(s).

16. A method of detecting the methylation profile of a sample *in vitro,* comprising:
(i) providing the sample and extracting nucleic acid;
(ii) detecting modification on CPG site(s) of a target sequence in the nucleic acid of (i), wherein the target sequence is the polynucleotide according to any of claims 1 to 2 or the polynucleotide converted therefrom according to any of claims 3 to 6.

17. The method according to claim 16, wherein, in step (3), the analysis methods comprise pyrosequencing, bisulfite conversion sequencing, method using methylation chip, qPCR, digital PCR, second generation sequencing, third generation sequencing, whole genome methylation sequencing, DNA enrichment detection, simplified bisulfite sequencing technology, HPLC, MassArray, methylation specific PCR, or their combination, as well as *in vitro* detection and *in vivo* tracer detection for the combined gene group of partial or all of the methylation sites in the sequence shown in SEQ ID NO:1.

18. The method according to claim 16, wherein, step (ii) comprises:
(1) treating the product of (i) to convert unmodified cytosine into uracil; preferably, the modification includes 5-methylation, 5-hydroxymethylation, 5-formylcytosine or 5-carboxylcytosine; preferably, treating the nucleic acid of step (i) with bisulfite;
(2) analyzing the modification of the target sequence in the nucleic acid treated by (1).
